Europäisches Patentamt

European Patent Office

Office européen des brevets

(19)

(11) Publication number: **0 067 171**
**B1**

(12) **EUROPEAN PATENT SPECIFICATION**

(45) Date of publication of patent specification: **29.01.86**

(21) Application number: **81903180.8**

(22) Date of filing: **09.11.81**

(86) International application number:
**PCT/US81/01500**

(87) International publication number:
**WO 82/02251 08.07.82 Gazette 82/17**

(51) Int. Cl.⁴: **G 01 N 33/28, G 01 N 31/22,**
**G 01 N 1/02, G 01 N 1/30**

(54) **METHOD AND ARTICLE FOR DETERMINING FREE ACID CONCENTRATION IN OILS.**

(30) Priority: **19.12.80 US 218356**

(43) Date of publication of application:
**22.12.82 Bulletin 82/51**

(45) Publication of the grant of the patent:
**29.01.86 Bulletin 86/05**

(84) Designated Contracting States:
**DE FR GB**

(56) References cited:
**GB-A- 963 794**
**US-A-2 245 557**
**US-A-2 770 530**
**US-A-2 852 693**
**US-A-3 006 735**
**US-A-3 016 292**
**US-A-3 067 015**
**US-A-3 193 356**
**US-A-3 238 020**
**US-A-3 259 463**
**US-A-3 510 263**
**US-A-3 543 570**

(73) Proprietor: **MINNESOTA MINING AND**
**MANUFACTURING COMPANY**
**3M Center, P.O. Box 33427**
**St. Paul, MN 55133 (US)**

(72) Inventor: **MLINAR, Jerry W.**
**P.O. Box 33427**
**St. Paul, MN 55133 (US)**
Inventor: **NEUMAYER, John J.**
**P.O. Box 33427**
**St. Paul, MN 55133 (US)**

(74) Representative: **Madgwick, Paul Roland et al**
**Ladas & Parry Isartorplatz 5**
**D-8000 München 2 (DE)**

(56) References cited:
**US-A-3 580 704**
**US-A-3 791 988**
**US-A-3 808 149**
**US-A-3 980 437**
**US-A-4 013 416**
**US-A-4 252 903**

Courier Press, Leamington Spa, England.

## Description

Technical field

This invention relates to a method and test strip for use in estimating the free acid content in oils such as cooking oil.

Background art

Great quantities of cooking oil and fats are utilized daily by cooks and food processors to prepare food. Typical cooking methods involve immersing the food to be cooked, e.g., in a wire basket, into a heated vat of cooking oil and permitting the food to remain therein until cooked. Cooking under such circumstances gives the food its own distinctive cooked flavor.

Unfortunately, as cooking oils and fats are used, there is a progressive build up of undesirable constituents such as free fatty acids. Cooking oils and fats used for frying foods are generally composed of glycerides with minor amounts of free fatty acids. The formation of additional free fatty acids has generally been attributed to the chemical break down of the glycerides to form fatty acids, mainly caused by hydrolysis and oxidation, producing short chain compounds such as aldehydes, ketones and acids. If degradation proceeds to any significant extent, the degradation products impart an unacceptable flavor to the cooked foods.

As the concentration of free fatty acid builds, the cooking oil progressively assumes undesirable characteristics such as fuming, darkening, smoking, forming gums and residues and imparting distinctive interfering flavors to the food being cooked.

Some cooks and food processors merely add new cooking oil or fat to the degraded material to extend the useful life of the cooking oil. While this is temporarily effective, the problem will be repeated as additional degradation takes place. Other cooks merely discard the entire batch of cooking oil or fat and replace it with a fresh supply thereby prolonging the time before additional corrective action must be taken. Many cooks and some food processors discard their cooking oil after a scheduled use time to preserve the integrity of the flavor of their cooked food notwithstanding the fact that degradation may not have occurred to an extent to even remotely interfere with the food. The latter method causes considerable waste and the unnecessary expenditure of considerable man hours, if the cooking oil or fat is discarded prematurely.

Various methods have been devised to quantitatively or qualitatively determine the degree of degradation and/or fatty acid content, but these generally have one or more problems associated therewith. One method involves monitoring the taste of the food and, when the food begins to assume an off taste, utilize that as a point at which the oil is replaced or rejuvenated. Since this method causes the flavor of the food to vary, it is undesirable. Other methods include monitoring the color of the oil, since it darkens with degradation, either by visually observing it or by using photoelectric devices to make the observation. These methods are undesirable because they generally require the removal and handling of small volumes of hot oil. Additionally, the photoelectric examination requires relatively expensive equipment which must be maintained and is subject to failure. Another method involves titrating small samples of the oil with a standard basic solution. The titration method is undesirable because it also requires removing samples of typically hot oil and also requires the preparation of standard titration solutions and the maintenance of certain laboratory equipment for use therewith, typically not found in most restaurants and some food processing facilities. Some test methods have been devised utilizing test papers, but they have generally been unacceptable because they have not been sufficiently accurate, are difficult to use, or give misleading results.

Illustrative of the prior art dealing with testing oils, fats and the like for degradation, fatty acid content, or similar purposes are the following U.S. Patents: Bergstrom et al (2,770,530), Seeman et al (3,030,190), Apter (3,615,226) and Elliott et al (2,953,439) disclose various "wet" methods of testing oils which require the removal of small amounts of oil from the main batch. Eiseman (3,238,020) discloses an oil test strip containing an indicator and an aliphatic polyhydroxy compound. Davis (3,420,635) discloses a fruit ripeness telltale formed of a sheet of plastic having thereon a color-changing composition including an acid-base indicator and a solid absorbent for $CO_2$ such as calcium hydroxide to detect when the fruit is ripended and a small amount of hygroscopic material such as calcium hydroxide to detect when the fruit is ripened and a small amount of hygroscopic material such as calcium chloride to insure the presence of moisture. Pickup et al (3,580,704) disclose testing free fatty acid content in oil with paper treated with an acid-indicator and a non-aqueous, nonvolatile liquid solvent in which the oil and indicator are soluble. Matsushita (4,098,575) discloses a test paper impregnated with potassium iodide to determine the peroxide value of oils in fats.

Summary of the invention

The present invention provides an article and a method for quantitatively estimating the concentration of free fatty acid in oils such as cooking oils or motor oil. The article is preferably in the form of a strip which can easily be submersed in the cooking oil and removed with a predetermined volume of oil imbibed thereon and therefore does not require removal of test samples of the oil, thereby avoiding liquid handling steps. The article of the invention provides a rapid, convenient means of determining the degree of fatty acid content in cooking oil, providing results in less than an hour.

The article of the present invention comprises a substantially nonreactive, neutral and nonbuffer- ing, porous absorbent support material having at least one portion thereof which contains a composition comprising an effective amount of acid-base indicator capable of changing color in a pH range on the order of 6—10, a predetermined amount of base compound reactive with free fatty acid and being present in an amount equivalent to a known concentration of free fatty acid, and about 30—99.5 parts by weight* of a substantially colorless, substantially neutral, humectant organic solvent which is substantially non-volatile under ambient conditions.

A preferred article of the invention includes spacially separated test areas, each of which contain the composition, but with varying amounts of base compound in each to provide the capability of identifying a range of concentrations of free fatty acid in the organic liquid. The method of the invention comprises contacting an organic liquid to be tested with a test article described above and observing any color change in the article after the passage of sufficient time.

Drawing

The invention may be further understood by reference to the drawing, wherein

Fig. 1 is a top plan of an embodiment of the article of the invention;

Fig. 2 is a side view of the article of Fig. 1;

Fig. 3 is a top plan view of another embodiment of the article of the invention having the capability of determining the fatty acid content over a range of concentrations:

Fig. 4 is a side view of the article of Fig. 3;

Fig. 5 is a top plan view of yet another embodiment of the article of the invention having a similar capability as the article of Figs. 3—4; and

Fig. 6 is a side view of the article of Fig. 5.

Description of the preferred embodiments

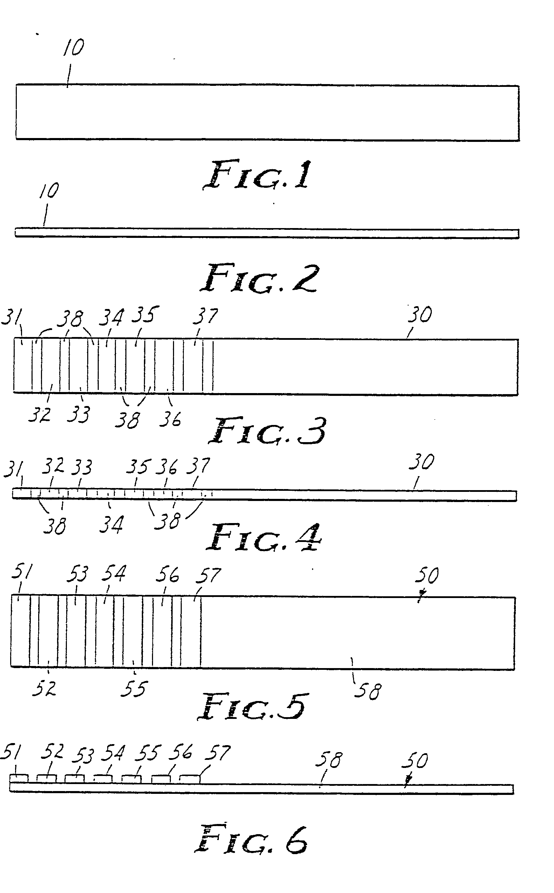

Referring now to Figs. 1—2, there is shown an article in the form of a strip 10 for estimating the free fatty acid content in organic liquids such as cooking oil. Strip 10 is formed of a substantially nonreactive, neutral and non-buffering support material which may be a woven or a nonwoven material. The preferred porous support material is formed of porous paper such as filter paper (e.g., Whatman's #1 filter paper). Other useful support materials include nonwoven glass filter material, open-celled foam and woven fabrics such as woven cotton cloth. The support material absorbs a controlled amount of oil in a given reasonable period of time, typically less than 1 minute, preferably less than 5 seconds. While the support material is preferably is in the form of an elongate strip, it may also have other forms, e.g., a tube, disc, rod or the like.

The support material is impregnated with a composition comprising an effective amount of acid-base indicator capable of changing color in a

_____

*based upon the weight of the non-volatile constituents of the solvent, indicator and the base compound.

pH range on the order of 6—10, a predetermined amount of base compound reactive with free fatty acid being present in an amount equivalent to its known concentration of free fatty acid present in the oil absorbed into the support material, and about 30—99.5% by weight (based upon the weight of the non-volatile constituents) of a substantially colorless, substantially neutral, humectant organic solvent which is substantially non-volatile under ambient conditions.

The article of Figs. 1—2 will be capable of changing color after a particular fatty acid content has been achieved in the organic liquid being tested and present in the support material. The amount of fatty acid at which the color change takes place is determined by the amount of base compound in the article. The amount of base should be adjusted to correlate to the desired concentration of free fatty acid. That is, if the amount of base is selected to provide a color change at 1% fatty acid, a color change will take place at 1% and higher concentrations of free fatty acid.

The preferred article according to the present invention has the capability of revealing a range of free fatty acid content, for example, in increments of 1% from 0 to 10%. Such articles are shown in Figs. 3—6. Referring to Figs. 3—5, there is shown an article in the form of strip 30 having test areas 31, 32, 33, 34, 35, 36 and 37, each containing the impregnant composition described above, except having varying amounts of base compound to reflect a varying range of free fatty acid content. The test areas are separated by a barrier stripe 38 which prevents migration of liquids from one area to the other to prevent interference between areas. Figs. 5—6 show yet another embodiment of the article of the invention in the form of strip 50 bearing test area segments 51—57, each containing varying amounts of base material to reflect a range of fatty acid content. The area segments are bits of base material which are impregnated as described above and adhered to a carrier strip 58. Other modifications of the article according to the present invention may be made without departing from the scope of the claims.

The support material may be formed of any substantially unreactive, i.e., pH neutral, non-buffering, and chemically non-reactive porous, absorbent woven, nonwoven, or the like material which has the capability of holding and retaining a sufficient amount of the active ingredients which will be contained therein. The support material is preferably, oil-absorbing and light colored or colorless. Suitable support materials may be formed of porous paper such as filter paper, preferably Whatman's #1 filter paper, nonwoven glass filters, open-celled foams, webs of polymeric microfibers, and woven cotton cloth.

The impregnated solution which is contained in the support material is formed of humectant solvent, indicator and base compound. The humectant solvent is a substantially non-volatile, substantially colorless, substantially neutral liquid that is soluble in water and capable of solubilizing the indicator and preferably the base compound. Suitable humectant solvents include mono and dihydroxy aliphatic polyethylene glycol compounds such as those available under the trade designation "Carbowax" 200, "Carbowax" 400 and "Carbowax" 600, dipolypropylene glycol methyl ether, and diethylene glycol ethyl ether and surfactants such as alkylphenoxy polyethoxy ethanols e.g., available under the trade designation "Triton" X-100. The humectant solvent comprises about 30—99.5% of the non-volatile content of the impregnant composition.

The base compound may be any organic or inorganic base compound with sufficient reactivity to react with the fatty acids or inorganic acids. Some examples of useful base compounds include sodium carbonate, sodium bicarbonate, potassium carbonate, potassium bicarbonate, tetramethyl guanidine, guanidine carbonate and amino silane (available under the trade designation "A-1100" silane from the Union Carbide Company). The amount of base compound may vary from 0.4% to 75% based upon the non-volatile constituents of the impregnant composition. The amount of base compound is controlled, depending upon the amount of oil imbibed upon the support, to provide in the article an amount which permits quantification of the amount of acid being measured.

The acid-base indicator may be any organic acid-base indicator dye or combination thereof which is capable of providing a color change, preferably a very distinct color change, in the pH range of 6—10. Suitable indicators include m-cresol purple, neutral red, thymol blue, phenol red and cresol red.

For strips which are capable of revealing a range of acid concentrations of the type shown in Figs. 3—4, a continuous stripe of barrier material is utilized to define test areas to prevent migration of the ingredients from one test area to another. The barrier material is any suitable material for providing this function. Examples of suitable barrier materials include polysiloxanes, polyacrylates and methacrylates, exposy resins and the like. Additionally, the barrier may be provided by the construction shown in Figs. 5—6 by employing a barrier type backing film 58 upon which the test area segments are adhered. Such backing materials may be formed of thermoplastic sheet material such as polyethylene, polypropylene or the like.

The simplest form of the article of the invention may be prepared by mixing the humectant solvent, base compound, indicator and additional volatile solvent such as water or organic solvent, if required, to provide a solution, and impregnating the support material with the solution to provide the desired composition within the base material and permitting the support material to dry.

Articles of the type depicted in Figs. 3—4 may be prepared by applying parallel separate stripes of barrier material to the base material and impregnating the areas defined between the stripes with impregnant compositions having varying amounts of base compound. The barrier stripes should be applied in a sufficient width to prevent migration between areas. Typical barrier widths are on the order of 1 to 10 mm. Segments of support material each having or subsequently being provided with varying amounts of base compound in the impregnant composition may be adhered to a barrier substrate by suitable adhesive or by heat lamination to provide the article depicted in Figs. 5—6. If the segments are to be heat laminated, it is preferred to impregnate the segments after lamination to avoid any heat induced alteration in their components. Additionally, an article according to the invention may be prepared by mixing the impregnant materials with paper or web-forming fibers and casting the web or paper from this mixture by conventional techniques. Other ways of producing the article of the invention will become apparent to those skilled in the art, once apprised of the present disclosure.

Examples

The invention is further illustrated by the following examples, wherein all parts are by weight, unless otherwise specified.

Example 1

An 11 cm diameter disc of Whatman's #1 filter paper was treated by spraying with a solution consisting of

| Ingredient | Parts |
|---|---|
| m-cresol purple indicator | 0.04 |
| ethylene glycol humectant non-volatile solvent available under the trade designation "Carbowax" 200 | 30.0 |
| sodium bicarbonate base compound | 0.78 |
| water volatile solvent | 69.18 |

The paper was coated to provide a wet coating weight of 15 mg per $cm^2$ and thereafter dried at room temperature to a constant weight of 3 mg per $cm^2$. A strip of the treated paper was dipped into oil containing known quantities of free fatty acid. The strip changed from blue to yellow approximately one hour after being dipped in oil containing free fatty acid to provide a concentration using an acid number in excess of 5. No color change was noted in the oil containing acid to provide an acid number less than 5.

Example 2

An article according to the present invention was prepared by laminating a 2 cm square piece of Whatman's #1 filter paper over the 2 cm square end portion of the polyethylene side of a 2 cm×10 cm strip of laminate film consisting of a layer of polyethylene adhered to a layer of polyester and available under the trade designation "Scotchpack" head sealable polyester film. Lamination of the paper to the film was accomplished with a "Robot" heat sealer (Model RTP-F available from Pack-Rite Machines) by heating the juxtaposed paper film assembly therein between jaws heated at 150°C, holding the assembly therein for 8 seconds at a jaw pressure of 0.24 kg per cm², to cause the paper to adhere to the polyethylene surface. The paper was then treated with the solution described in Example 1 to provide a wet coating weight of 15 mg per cm² and dried at room temperature to a constant weight to provide a dry coating weight of 3 mg per cm². The resultant strip performed substantially the same as the strip described in Example 1.

Example 3

An article of the type depicted in Figs. 5 and 6 according to the present invention having multiple test areas corresponding to acid numbers of 1, 3, 5 and 7 was produced by laminating four 0.5 cm by 1 cm pieces of Whatman's #1 filter paper on the polyethylene side of a 1 cm×10 cm strip of the polyethylene:polyester laminate film described in Example 2 with the four strips of paper being separated by 0.5 cm and being parallel on their 1 cm sides with their 0.5 cm sides aligned along the 10 cm sides of the laminate. The assembly was laminated in the heat sealer described in Example 2 at 150°C for 10 seconds at a pressure of 0.24 kg per cm². The four segments of filter paper were treated respectively with the following solutions

| Ingredient | Parts | Parts | Parts | Parts |
|---|---|---|---|---|
| Acid No. | 1 | 3 | 5 | 7 |
| m-cresol purple indicator | 0.04 | 0.04 | 0.04 | 0.04 |
| ethylene glycol humectant solvent available under the trade designation "Carbowax" 200 | 30.0 · | 30.0 | 30.0 | 30.0 |
| sodium bicarbonate base compound | 0.275 | 0.5 | 0.78 | 1.07 |
| water (volatile solvent) | 69.69 | 69.46 | 69.18 | 68.88 |

Each of the coating weights was approximately 15 mg per cm² wet, drying to a constant 3 mg per cm² weight at room temperature. The resultant dried strip was dipped into a test oil sample containing fatty acid concentration of less than acid number one. No color change in the strip was observed, i.e., all of the zones remained blue. A second strip was dipped into oil containing fatty acid to provide an acid number greater than 1 but less than 3. A color change from blue to yellow in the test area treated to correspond to an acid number of 1 was observed, but no color change in the remaining treated test areas indicating acid numbers of 3 or more were noted. The remaining test areas were found to perform in a similar manner when the free fatty acid concentration was increased.

Example 4

A preferred article depicted in Figs. 3—4 of the drawing according to the present invention having the capability of detecting the range of fatty acid concentrations corresponding to acids number 1, 3, 5 and 7 was prepared. A 1 cm×10 cm strip of Whatman's #1 filter paper was provided with barrier stripes consisting of a two part silicone resin available from the Dow Corning Company as "Dielectric Gel" and applied by utilizing a 1 cc hypodermic syringe fitted with a 25 gauge needle to provide parallel stripes on the strip of filter paper parallel to the 1 cm ends 2 mm wide and separated by approximately 4 mm to provide four 4 mm×10 mm zones of untreated paper. Each zone was treated, respectively, with the four impregnant compositions described in the previous example to provide test areas having respectively the capability of detecting acid numbers 1, 3, 5 and 7. The test strip performed substantially the same as described in Example 3.

**Claims**

1. An article for quantitatively determining the concentration of free fatty acid in an oil, said article comprising a non-reactive, neutral, porous and absorbent, support material having at least one portion thereof which is impregnated with a composition characterized by comprising

(a) an effective amount of acid-base indicator capable of changing color in a pH range on the order of 6—10;

(b) a predetermined amount of base compound reactive with fatty acid absorbed in said support and being present in an amount equivalent to a known concentration of free fatty acid; and

(c) about 30—99.5 parts by weight based upon the total weight of the non-volatile constituents of (a), (b) and (c) of a colorless, neutral, humectant organic solvent which is non-volatile under ambient conditions.

2. The article of claim 1 wherein said humectant solvent is characterized by being selected from the group consisting of hydroxy aliphatic polyethylene glycol compounds and alkylphenoxy polyethoxy ethanols.

3. The article of claim 1 wherein said base compound is characterized by being selected from the group consisting of sodium carbonate, sodium bicarbonate, potassium carbonate, potassium bicarbonate, tetramethyl guanidine, guanidine carbonate and amino silane.

4. The article of claim 1 wherein said indicator is characterized by being selected from the group consisting of m-cresol purple, neutral red, thymol blue, phenol red and cresol red.

5. The article of claim 1 wherein said support material is characterized by having a plurality of test areas, each containing said composition but with varying predetermined amounts of said base compound in each said test areas to provide amounts equivalent to a range of known concentrations of free acids.

6. The article of claim 5 wherein said test areas are characterized by being separated by barrier stripes which prevent the migration of ingredients from one test area to an adjacent test area.

7. The article of claim 5 wherein said test areas are characterized by being spacially separated and adhered to a carrier strip formed of a barrier material which prevents migration of ingredients from one of said test areas to another.

8. A method of quantitatively determining the concentration of free fatty acid in an oil comprising

(a) contacting the oil to be tested with a test article comprising a non-reactive neutral, porous, absorbent and non-buffering support material containing an active composition; and

(b) observing any color change in said article characterized by said active composition comprising

(1) an effective amount of acid-base indicator capable of changing color in a pH range on the order of 6—10;

(2) a predetermined amount of base compound reactive with free fatty acid and being present in an amount equivalent to a known concentration of free fatty acid absorbed in said support material; and

(3) about 30—99.5 parts by weight based upon the total weight of the non-volatile constituents of (1), (2) and (3) of a neutral, humectant organic solvent which is non-volatile under ambient conditions.

**Patentansprüche**

1. Gegenstand zur quantitativen Bestimmung der Konzentration von freien Fettsäuren in einem Öl, mit einem nicht reaktionsfähigen, neutralen, porösen und absorptionsfähigen Trägermaterial, das mindestens in einem Teil mit einer Zusammensetzung imprägniert ist, dadurch gekennzeichnet, daß die Zusammensetzung enthält:

(a) Ein wirksame Menge eines Säure-Basen-Indikators, der bei einem pH-Wert im Bereich von 6 bis 10 zu einer Farbveränderung befähigt ist;

(b) eine vorherbestimmte Menge einer basischen Verbindung, die zur Reaktion mit von dem Trägermaterial absorbierter Fettsäure befähigt und in einer Menge vorhanden ist, die einer bekannten Konzentration von freien Fettsäuren äquivalent ist; und

(c) ein Feuchthaltemittel in Form eines neutralen organischen Lösungsmittels, das unter Umgebungsbedingungen nichtflüchtig und das in einer Menge von 30 bis 99,5 Gewichtsteilen des Gesamtgewichtes der nichtflüchtigen Bestandteile von (a), (b) und (c) vorhanden ist.

2. Gegenstand nach Anspruch 1, dadurch gekennzeichnet, daß das Feuchthaltemittel in Form des Lösungsmittels aus der Gruppe ausgewählt ist, die aus den hydroxyaliphatischen Polyethylenglykolverbindungen und den Alkylphenoxypolyethoxyethanolen besteht.

3. Gegenstand nach Anspruch 1, dadurch gekennzeichnet, daß die basische Verbindung aus der Gruppe ausgewählt ist, die aus Natriumcarbonat, Natriumbicarbonat, Kaliumcarbonat, Kaliumbicarbonat, Tetramethylguanidin, Guanidincarbonat und Aminosilan besteht.

4. Gegenstand nach Anspruch 1, dadurch gekennzeichnet, daß der Indikator aus der Gruppe ausgewählt ist, die aus m-Kresolpurpur, Neutralrot, Thymolblau, Phenolrot und Kresolrot besteht.

5. Gegenstand nach Anspruch 1, dadurch gekennzeichnet, daß das Trägermaterial mehrere Prüfbereiche besitzt, die alle die genannte Zusammensetzung haben, aber die basische Verbindung in unterschiedlichen vorbestimmten Mengen enthalten, die benannten Konzentrationen von freien Säuren in einem Bereich von Konzentration äquivalent sind.

6. Gegenstand nach Anspruch 5, dadurch gekennzeichnet, daß die Prüfbereiche voneinander durch Sperrstreifen getrennt sind, die eine Wanderung von Bestandteilen aus einem Prüfbereich in einen benachbarten Prüfbereich verhindern.

7. Gegenstand nach Anspruch 5, dadurch gekennzeichnet, daß die Prüfbereiche räumlich voneinander getrennt und haftfest mit einem Trägerstreifen verbunden sind, der aus einem Sperrmaterial besteht, das eine Wanderung von Bestandteilen aus einem Prüfbereich in einen anderen verbindert.

8. Verfahren zum quantitativen Bestimmen der Konzentration von freien Fettsäure in einem Öl, in dem

(a) das zu prüfende Öl mit einem Prüfgegenstand in Berührung gebracht wird, der ein nicht reaktionsfähiges, neutrales poröses, absorptionsfähiges, nichtpufferndes Trägermaterial besitzt, das eine aktive Zusammensetzung enthält, und

(b) eine etwaige Farbveränderung in dem Gegenstand festgestellt wird, dadurch gekennzeichnet, daß die genannten aktive Zusammensetzung enthält:

(1) Eine wirksame Menge eines Säure-Basen-Indikators, der bei einem pH-Wert im Bereich von 6 bis 10 zu einer Farbveränderung befähigt ist;

(2) eine vorherbestimmte Menge einer basischen Verbindung, die zur Reaktion mit absorbierter Fettsäure befähigt und in einer Menge vorhanden ist, die einer bekannten Konzentration von in dem Gegenstand absorbierten, freien Fettsäuren äquivalent ist; und

(3) ein Feuchthaltemittel in Form eines neutralen organischen Lösungsmittels, das unter Umgebungsbedingungen nichtflüchtig und das in einer Menge von 30 bis 99,5 Gewichtsteilen des Gesamtgewichtes der nichtflüchtigen Bestandteile von (1), (2), und (3) vorhanden ist.

**Revendications**

1. Article pour la détermination quantitative de la concentration en acide gras libre d'une huile, le dit article comprenant une matière support poreuse absorbante, inerte et neutre, dont au moins une portion est imprégnée d'une composition, caractérisé en ce que ladite composition comprend:

a) une quantité efficace d'un indicateur acido-basique capable de changer de couleur dans une gamme de pH de l'ordre de 6 à 10;

b) une quantité prédéterminée de compose basique réagissant avec un acide gras absorbé dans le dit support et étant présent et une quantité équivalant à une concentration connue d'acide gras libre; et

c) environ 30 à 99,5 parties en poids, par rapport au poids total des constituants non volatils de (a), (b) et (c), d'un solvant organique humectant incolore et neutre qui n'est pas volatil dans les conditions ambiantes.

2. Article selon la revendication 1, dans lequel le dit solvant humectant est caractérisé en ce qu'il est choisi parmi l'ensemble constitué par les composés aliphatiques hydroxylés du poly-éthylèneglycol et les alkylphénoxy-polyéthoxy-éthanols.

3. Article selon la revendication 1, dans lequel le dit composé basique est caractérisé en ce qu'il est choisi parmi l'ensemble constitué par les carbonate de sodium, bicarbonate de sodium, carbonate de potassium, bicarbonate de potassium, tétraméthylguanidine, carbonate de guanidine, et aminosilane.

4. Article selon la revendication 1, dans lequel le dit indicateur est caractérisé en ce qu'il est choisi parmi l'ensemble constitué par le pourpre de m-crésol, le rouge neutre, le bleu de thymol, le rouge de phénol et le rouge de crésol.

5. Article selon la revendication 1, dans lequel la dite matière support est caractérisée en ce qu'elle a plusieurs zones d'essai contenant chacune la dite composition mais avec des quantités prédéterminées variables dudit composé basique dans chacune des dites zones d'essai pour fournir des quantités équivalent à une gamme de concentrations connues d'acides libres.

6. Article selon la revendication 5, dans lequel les dites zones d'essai sont caractérisées en ce qu'elle sont séparées par des bandes d'arrêt qui empêchent la migration des ingrédients d'une zone d'essai à une zone d'essai adjacente.

7. Article selon la revendication 5, dans lequel les zones d'essai sont caractérisées en ce qu'elles sont séparées dans l'espace et adhèrent à une bandelette support faite d'une matière d'arrêt qui empéche la migration des ingrédients d'une des dites zones d'essai à l'autre.

8. Procédé de détermination quantitative de la concentration d'acide gras libre dans une huile, comprenant:

a) le contact de l'huile à étudier avec un article d'essai comprenant une matière support inerte, neutre, poreuse, absorbante et non tamponnante, contenant une composition active; et

b) l'observation de tout changement de couleur dudit article, caractérisé en ce que la dite composition active comprend:

(1) une quantité efficace d'un indicateur acido-basique capable de changer de couleur dans une gamme de pH de l'ordre de 6 à 10;

(2) une quantité prédéterminée d'un composé basique réagissant avec l'acide gras libre et qui est présent en une quantité équivalant à une concentration connue d'acide gras libre absorbé dans ladite matière support; et

(3) environ 30 à 99,5 parties en poids du poids total des constituants non volatils de (1), (2) et (3) d'un solvant humectant organique neutre qui n'est pas volatil dans les conditions ambiantes.

10

*FIG.1*

10

*FIG.2*

31    38  34 35    37                           30

32  33   38  36   *FIG.3*

31  32 33    35 36 37                          30

38     34    38   *FIG.4*

51    53 54   56 57                          50

52      55              58   *FIG.5*

51  52 53 54 55 56 57      58    50

*FIG. 6*